(19) Europäisches Patentamt
European Patent Office
Office européen des brevets

(11) Publication number: 0 312 234
A2

(12) # EUROPEAN PATENT APPLICATION

(21) Application number: 88309218.1

(51) Int. Cl.[4]: A61K 7/06

(22) Date of filing: 04.10.88

(30) Priority: 16.10.87 GB 8724285

(43) Date of publication of application:
19.04.89 Bulletin 89/16

(84) Designated Contracting States:
AT BE CH DE FR GB GR IT LI LU NL SE

(71) Applicant: THE PROCTER & GAMBLE COMPANY
One Procter & Gamble Plaza
Cincinnati Ohio 45202(US)

(72) Inventor: Gummer, Christopher Lawrence
High Croft Dorking Road
Chilworth Surrey GU4 8RR(GB)

(74) Representative: Brooks, Maxim Courtney et al
Procter & Gamble (NTC) Limited Whitley Road Longbenton
Newcastle-upon-Tyne NE12 9TS(GB)

(54) **Shampoo compositions.**

(57) Antidandruff shampoo compositions comprising a synthetic surfactant; a dispersed insoluble, non-volatile silicone; a long chain acyl derivative or amide oxide; a pyridinethione antidandruff agent; a hydroxypyridone and/or imidazolyl ketone antidandruff agent; and water. The compositions combine excellent hair conditioning effectiveness, antidandruff efficacy and product stability.

EP 0 312 234 A2

# SHAMPOO COMPOSITIONS

## TECHNICAL FIELD

The present invention relates to shampoo compositions. In particular, it relates to shampoo compositions having simultaneous hair conditioning and antidandruff efficacy together with improved formulation characteristics.

## BACKGROUND

Antidandruff shampoos are well known in the art and rely on a number of different agents for their antidandruff effectiveness. However shampooing with an antidandruff shampoo is done not only to relieve a dandruff condition but also to clean the hair.

Human hair becomes soiled due to its contact with the surrounding atmosphere and, to a greater extent, from sebum secreted by the head. The build-up of the sebum causes the hair to have a dirty feel and an unattractive appearance. The soiling of the hair necessitates it being shampooed with frequent regularity.

Shampooing the hair cleans by removing excess soil and sebum. However, the shampooing process has disadvantages in that the hair is left in a wet, tangled and generally unmanageable state. A variety of approaches have been developed to alleviate the after-shampoo problems. These range from the inclusion of hair conditioning aids in shampoos to post-shampoo application of hair conditioners, i.e., hair rinses. Hair rinses typically work by depositing a polymeric film or other material onto the hair. However, such solutions to a very prevalent problem have not been fully satisfactory. For one thing, hair rinses must be applied in a separate step following the shampooing, left on the hair for a length of time, and rinsed with fresh water. This, of course, is time consuming and inconvenient.

While shampoos, both antidandruff and regular, have been disclosed which contain conditioning aids, they have not been totally satisfactory for a variety of reasons. One problem relates to compatibility problems between good cleaning anionic surfactants and the fatty cationic agents which are good conditioning agents. Another problem in that shampoo conditioning performance can be adversely affected by the antidandruff agent itself, leading either to loss in hair conditioning effectiveness (diminished softness, wet combing characteristics) or to side-effects such as hair greasiness, rapid re-soiling etc.

The use of silicones in shampoo compositions has been disclosed in a number of different publications including US-A- 2,826,551, US-A-3,964,500, US-A-4,364,837 and GB-A-849,433. While these patents disclose silicone containing compositions, they do not provide answers to all of the problems encountered in making a product which is totally satisfactory from the viewpoint of providing optimum hair conditioning and antidandruff efficacy as well as good formulation stability and in-use characteristics.

## SUMMARY OF THE INVENTION

According to the present invention, there is provided an antidandruff shampoo composition comprising:

(a) from about 5% to about 70% by weight of a synthetic surfactant;

(b) from about 0.01% to about 10% by weight of a dispersed, insoluble non-volatile silicone;

(c) from about 0.5% to about 5% by weight of a long chain ($C_{16}$-$C_{22}$) acyl derivative or a long chain ($C_{16}$-$C_{22}$) amide oxide;

(d) from about 0.1% to about 3% of a first antidandruff agent which is a 1-hydroxy-2-pyridinethione salt (I);

(e) from about 0.1% to about 3% of a second antidandruff agent selected from

i) hydroxypyridones having the general formula II, or a salt thereof:

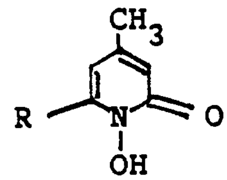

II

wherein R is an aliphatic or aromatic moiety having a π factor of at least 1.3;

ii) imidazolyl ketone derivatives of the general formula III

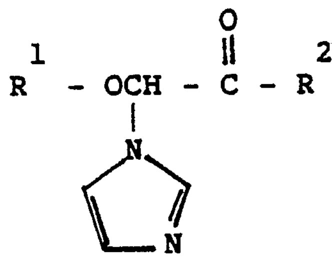

III

wherein $R^1$ and $R^2$ are independently selected from optionally substituted alkyl, alkenyl, alkynyl, cycloalkyl, aryl and alkaryl groups and

iii) mixtures of (i) and (ii); and

f) water

Unless otherwise indicated, all percentages and ratios herein are by weight.

An essential component of the antidandruff shampoo compositions of the invention is a surfactant. The surfactant can be any of a wide variety of synthetic surfactants selected from anionic, nonionic, amphoteric and zwitterionic surfactants. The surfactant component usually comprises from about 5% to about 70% by weight of the composition, preferably from about 10% to about 30%, most preferably from about 10% to about 22%.

Synthetic anionic surfactants can be exemplified by the alkali metal salts of organic sulfuric reaction products having in their molecular structure an alkyl radical containing from 8 - 22 carbon atoms and a sulfonic acid or sulfuric acid ester radical (included in the term alkyl is the alkyl portion of higher acyl radicals). Preferred are the sodium, ammonium, potassium or triethanolamine alkyl sulfates, especially those obtained by sulfating the higher alcohols ($C_8$ - $C_{18}$ carbon atoms), sodium coconut oil fatty acid monoglyceride sulfates and sulfonates; sodium or potassium salts of sulfuric acid esters of the reaction product of 1 mole of a higher fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms, sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and others known in the art.

Nonionic surfactants can be broadly defined as compounds produced by the condensation of alkylene oxide groups (hydrophilic in nature) with an organic hydrophobic compound, which may be aliphatic or alkyl aromatic in nature. Examples of preferred classes of nonionic surfactants are:

1. The polyethylene oxide condensates of alkyl phenols, e.g., the condensation products of alkyl phenols having an alkyl group containing from about 6 to 12 carbon atoms in either a straight chain or branched chain configuration, with ethylene oxide, the said ethylene oxide being present in amounts equal to 10 to 60 moles of ethylene oxide per mole of alkyl phenol. The alkyl substituent in such compounds may be derived from polymerixed propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from about 40% to about 80% polyoxyethylene by weight and having a molecular weight of from

about 5,000 to about 11,000 resulting from the reaction of ethylene oxide groups with a hydrophobic base constituted of the reaction product of ethylene diamine and excess propylene oxide, said base having a molecular weight of the order of 2,500 to 3,000, are satisfactory.

3. The condensation product of aliphatic alcohols having from 8 to 18 carbon atoms, in either straight chain or branched chain configuration, with ethylene oxide, e.g., a coconutalcohol ethylene oxide condensate having from 10 to 30 moles of ethylene oxide per mole of coconut alcohol, the coconut alcohol fraction having from 10 to 14 carbon atoms.

4. Long chain tertiary amine oxides corresponding to the following general formula:

$R_1R_2R_3N{\rightarrow}O$

wherein $R_1$ contains an alkyl, alkenyl or monohydroxy alkyl radical of from about 8 to about 18 carbon atoms, from 0 to about 10 ethylene oxide moieties, and from 0 to 1 glyceryl moiety, and $R_2$ and $R_3$ contain from 1 to about 3 carbon atoms and from 0 to about 1 hydroxy group, e.g., methyl, ethyl, propyl, hydroxy ethyl, or hydroxy propyl radicals. The arrow in the formula is a conventional representation of a semipolar bond. Examples of amine oxides suitable for use in this invention include dimethyldodecylamine oxide, oleyldi(2-hydroxyethyl)amine oxide, dimethyloctylamine oxide, dimethyldecylamine oxide, dimethyltetradecylamine oxide, 3,6,9-trioxaheptadecyldiethylamine oxide, di(2-hydroxyethyl)-tetradecylamine oxide, 2-dodecoxyethyldimethylamine oxide, 3-dodeoxycy-2-hydroxypropyldi(3-hydroxypropyl)amine oxide, dimethylhexadecylamine oxide.

Zwittericnic surfactants can be exemplified by those which can be broadly described as derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

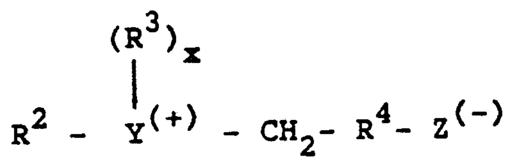

wherein $R^2$ contains an alkyl, alkenyl, or hydroxy alkyl radical of from about 8 to about 18 carbon atoms, from 0 to about 10 ethylene oxide moieties and from 0 to 1 glyceryl moiety; Y is selected from nitrogen, phosphorus, and sulfur atoms, $R^3$ is an alkyl or monohydroxyalkyl group containing 1 to about 3 carbon atoms; x is 1 when Y is a sulfur atom and 2 when Y is a nitrogen or phosphorus atom; $R^4$ is an alkylene or hydroxyalkylene of from 1 to about 4 carbon atoms and Z is a radical selected from carboxylate, sulfonate, sulfate, phosphonate, and phosphate groups.

Examples of zwitterionic surfactants useful herein include the high alkyl betaines such as coco dimethyl carboxymethyl betaine, lauryl dimethyl carboxymethyl betaine, lauryl dimethyl alphacarboxyethyl betaine, cetyl dimethyl carboxymethyl betaine, lauryl bis-(2-hydroxyethyl)carboxy methyl betaine, stearyl bis-(2-hydroxypropyl)carboxymethyl betaine, oleyl dimethyl gamma-carboxypropyl betaine, lauryl bis-(2-hydroxypropyl) alpha-carboxypropyl betaine, lauryl bis-(2-hydroxypropyl) alpha-carboxyethyl betaine, etc. The sulfobetaines may be represented by coco dimethyl sulfopropyl betaine, stearyl dimethyl sulfopropyl betaine, lauryl dimethyl sulfoethyl betaine, lauryl bis-(2-hydroxyethyl) sulfopropyl betaine and the like; amido betaines and amidosulfobetaines, wherein the $RCONH(CH_2)_3$ radical is attached to the nitrogen atom of the betaine are also useful in this invention.

Examples of amphoteric surfactants which can be used in the compositions of the present invention can be broadly described as derivatives of aliphatic secondary and tertiary amines in which the aliphatic radical can be straight chain or branched and wherein one of the aliphatic substituents contains from about 8 to about 18 carbon atoms and one contains an anionic water solubilizing groups, e.g., carboxy, sulfonate, sulfate, phosphate, or phosphonate. Examples of compounds falling within this definition are sodium 3-dodecylaminopropionate, sodium 3-dodecylaminopropane sulfonate, N-alkyltaurines such as the one prepared by reacting dodecylamine with sodium isethionate according to the teaching of US-A-2,658,072, N-higher alkyl aspartic acids such as those produced according to the teaching of US-A-2,438,091, and the products sold under the trade name "Miranol" and described in US-A-2,528,378.

Many additional nonsoap surfactants are described in McCUTCHEON'S, DETERGENTS AND EMULSIFIERS, 1979 ANNUAL, published by Allured Publishing Corporation.

The above-mentioned surfactants can be used alone or in combination in the shampoo compositions of the present invention. The anionic surfactants, particularly the alkyl sulfates, the ethoxylated alkyl sulfates and mixtures thereof are preferred for use herein. An especially preferred surfactant system of this type is a mixture of lauryl sulfate and ethoxylated lauryl sulfate surfactants in a molar ratio of less than about 1:3.

The non-volatile silicone component of the present composition generally has a boiling point at atmospheric Pressure in excess of about 300°C and is present at a level of from about 0.1% to about 10%, preferably form about 0.5% to about 5% by weight. The silicone should be insoluble in the shampoo matrix whereby the silicone takes the form of a particulate silicone dispersion Suitable non-volatile silicones can be in either fluid or gum form, silicones in fluid form being represented by polyalkyl siloxanes, polyaryl siloxanes, polyalkylaryl siloxanes and polyether siloxane copolymers. Mixtures of these fluids may also be used and are preferred in certain executions.

The essentially non-volatile polyalkyl siloxane fluids that may be used include, for example, polydimethyl siloxanes with viscosities ranging from about 5 to about 600,000 centistokes at 25°C. These siloxanes are available, for example, from the General Electric Company as the Viscasil series and from Dow Corning as the Dow Corning 200 series. The viscosity can be measured by means of a glass capillary viscometer as set forth in Dow Corning Corporate Test Method CTMOOO4, July 20, 1970. Preferably the viscosity ranges from about 350 centistokes to about 100,000 centistokes.

The essentially non-volatile polyalkylaryl siloxane fluids that may be used include, for example, polymethylphenylsiloxanes having viscosities of about 15 to about 30,000 centistokes at 25°C. These siloxanes are available, for example, from the General Electric Company as SF 1075 methyl phenyl fluid or from Dow Corning as 556 Cosmetic Grade Fluid.

The essentially non-volatile polyether siloxane copolymer that may be used is, for example, a polypropylene oxide modified dimethylpolysiloxane (e.g., Dow Corning DC-1248) although ethylene oxide or mixtures of ethylene oxide and propylene oxide may also be used.

References disclosing suitable silicone fluids include US-A-2,826,551, US-A-3,964,500, US-A-4,364,837 and Silicon Compounds distributed by Petrarch Systems, Inc., 1984. This reference provides a very good listing of suitable silicone materials.

Another silicone material found especially useful in the present compositions to provide good dry combing is a silicone gum. Silicone gums described by Petrarch and others including US-A-4,152,416, and Noll, Walter, Chemistry and Technology of Silicones, New York: Academic Press 1968. Also describing silicone gums are General Electric Silicone Rubber Product Data Sheets SE 30, Se 33, SE 54 and SE 76. "Silicone gum" materials denote high molecular weight polydiorganosiloxanes having a mass molecular weight of from about 200,000 to about 1,000,000. Specific examples include polydimethylsiloxane, polydimethylsiloxane/methylvinylsiloxane copolymer, polydimethylsiloxane/diphenylmethylvinylsiloxane copolymer and mixtures thereof.

The suspending agent useful in the present compositions can be any of several long chain acyl derivative materials or mixtures of such materials. Included are ethylene glycol esters of fatty acids having from about 16 to about 22 carbon atoms. Preferred are the ethylene glycol stearates, both mono and distearate, but particularly the distearate containing less than about 7% of the mono stearate. Other suspending agents found useful are alkanolamides of fatty acids, having from about 16 to 22 carbon atoms, preferably from 16 to 18 carbon atoms. Preferred alkanolamides are stearic monoethanolamide, stearic diethanolamide, stearic monoisopropanolamide and stearic monethanolamide stearate. Other long chain acyl derivatives include long chain esters of long chain fatty acids (e.g., stearyl stearate, cetyl palmitate, etc.); glyceryl esters (e.g., glyceryl distearate) and long chain esters of long chain alkanolamides (e.g., stearamide DEA distearate, stearamide MEA stearate).

Still other suitable suspending agents are alkyl ($C_{16-22}$) dimethylamine oxides such as stearyl dimethylamide oxide. If the compositions contain an amide oxide or a long chain acyl derivative as a surfactant, the suspending function could also be provided and additional suspending agent may not be needed if the level of those materials are at least the minimum level given below.

The suspending agent is present at a level of from about 0.50% to about 5.0%, preferably from about 0.5% to about 3.0%. The suspending agent serves to assist in suspending the silicone material and the pyridinethione salt and may give pearlescence to the product. Mixtures of suspending agents are also suitable for use in the compositions of this invention.

The pyridinethione salt crystals used in the present composition is preferably in the form of flat platelets. Such crystals are heavy metal, magnesium or aluminium salts of 1-hydroxy-2-pyridinethione which has the following structural formula in tautomeric form, the sulfur being attached to the No. 2 position in the

pyridine ring.

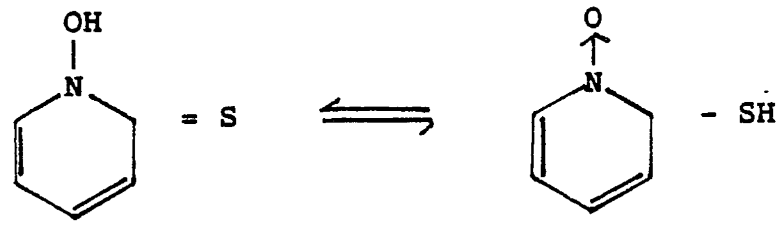

The metal salts represent substitution of the metal for the hydrogen of one of the tautomeric forms. Depending, of course, on the valence of the metal involved there may be more than one of the pyridinethione rings in the compound. Suitable heavy metals include zinc, tin, cadmium and zirconium.

The preferred pyridinethione salt crystals are predominantly flat platelets which have a mean sphericity of less than about 0.65, preferably between about 0.20 and about 0.65, and a median particle size of at least about 2µm diameter, expressed as the median equivalent diameter of a sphere of equal volume. It is preferred that the mean individual particle size be not greater than about 25µm, measured on the same basis. The median diameters are on a mass basis with 50% of the mass of particles falling on either side of the value given.

The diameter of a sphere of equivalent volume for a particle can be determined by a variety of sedimentation techniques which are based on Stokes' Law for the settling velocity of a particle in a fluid. Such techniques are described in Stockham, J.D. and Fochtman, E.G., Particle Size Analysis, Ann Arbor Science, 1978. An approach for determining the median equivalent spherical diameter based on volume, $\bar{d}_v$, is shown in EP-A-34, 385, Example II.

The sphericity of a particle is also described by Stockham and Fochtman at page 113 as $(d_v/d_s)^2$, where $d_v$ is the diameter of a sphere of equivalent volume, supra, and $d_s$ is the diameter of a sphere of equivalent area. As used herein, however, the mean sphericity is $(\bar{d}_v/\bar{d}_s)^2$, or the surface area of spheres having equivalent volume distribution divided by the actual surface area of particles as measured. A technique for determining actual surface area is shown in the examples using the BET technique described by Stockham and Fochtman at page 122.

The pyridionethione salt is used at a level of from about 0.1% to about 3%, preferably from about 0.5% to about 2%.

The compositions of the invention also contain a second antidandruff agent selected from hydroxypyridones, imidazolyl ketones and mixtures thereof. The second antidandruff component comprises a total of from about 0.1% to about 3% of by weight of the compositions, preferably from about 0.5% to about 2% by weight.

The hydroxypyridone component (II) can generally be described as 1-hydroxy-4-methyl-(IH)-pyridones having an aliphatic or aromatic moiety (R) at the 6 position thereof, wherein R has a $\pi$ factor of at least 1.3, preferably from 2 to 6, more preferably from 3 to 5.5. The $\pi$ factor is a measure of the lipophilicity/hydrophilicity of the substituent and is defined in detail in the paper by W. Dittmar, E. Druckrey and H. Urbach, J. Med. Chem. 17(7), 753-6(1974) and references cited therein.

In structural terms, preferred R substituents are selected from linear and branched $C_3$-$C_{11}$, preferably $C_6$-$C_{11}$ alkyl and alkenyl groups, $C_5$-$C_8$ cycloalkyl groups, and aryl groups. In the above, cyclic moieties can also be substituted with one or more alkyl or alkenyl groups up to $C_4$. The R groups can further be substituted with halogen atoms. Of the above, preferred R moieties are cyclohexyl and 2,4,4-trimethyl pentyl, the latter being highly preferred.

The above mentioned compounds can be used both in the free form and as salts, for example, salts with organic bases or inorganic cations. Low molecular weight alkanolamines are especially preferred organic bases, for example triethanolamine.

The imidazolyl ketone component of the present compositions has the general formula III set out hereinabove. Preferably $R^1$ and $R_2$ are independently selected from optionally substituted $C_1$-$C_8$ alkyl, alkenyl and alkynyl; optionally substituted aryl and $C_{1-4}$ alkaryl; and optionally substituted $C_5$-$C_7$ cycloalkyl and cycloalkenyl groups. Of the above, $R^1$ is preferably an optionally halo substituted (e.g. chloro or bromo or fluoro) aryl or $C_1$-$C_4$ alkaryl group and $R^2$ is preferably a $C_1$–$C_8$ straight chain or branched alkyl or alkenyl group. The highly prefered imidazolyl ketone is 1-(p-chlorophenoxy)-1-(1H-imidazol-1-yl)-3,3-dimethyl -2-butanone.

Water is the last essential component of the present invention and is present at a level of at least about

20% and preferably from about 60% to about 85%.

The shampoos herein can contain a variety of non-essential optional components suitable for rendering such compositions more acceptable. Such conventional optional ingredients are well known to those skilled in the art, e.g., preservatives such as benzyl alcohol, methyl paraben, propyl paraben and imidazolidinyl urea; quaternary ammonium surfactants such as cetyl trimethyl ammonium chloride, lauryl trimethyl ammonium chloride, tricetyl methyl ammonium chloride, stearyldimethyl benzyl ammonium chloride, and di-(partially hydrogenated tallow) dimethylammonium chloride; thickeners and viscosity modifiers such as mono- and diethanolamides of $C_8$-$C_{14}$ fatty acids (e.g., coconut monoethanolamide, lauric or coconut diethanolamide PEG 3 lauramide), block polymers of ethylene oxide and propylene oxide such as Pluronic F88 offered by BASF Wyandotte, sodium chloride, sodium sulfate, polyvinyl alcohol, and ethyl alcohol; pH adjusting agents such as citric acid, succinic acid, phosphoric acid, sodium hydroxide, sodium carbonate, etc.; perfumes; dyes; and sequestering agents such as disodium ethylenediamine tetraacetate. Such agents generally are used individually at a level of from about 0.1% to about 10%, preferably from about 0.5% to about 5.0% by weight of the composition, although the quaternary ammonium surfactants should be limited to levels of less than 4% and preferably less than 2%.

The pH of the present compositions is not critical and may be in the range of from 2 to about 10, preferably from about 4 to about 7.

One method for manufacturing the present composition is described below.

A premix of the silicone and surfactant components is first prepared and a second premix containing all remaining ingredients except the pyridinethione salt is then prepared and heated to about 72°C. The two premixes are then combined and stirred thoroughly for about 10 minutes at 72°C before pumping through a high shear mill and then through a heat exchanger to cool to about 27°C. The pyridinethione salt is then added at 27°C and is thoroughly mixed.

The high shear mill is used to achieve adequate dispersion of the silicone fluid. This is achieved by having the average particle size of about 10µm or less.

In the cooling step, the acyl derivative is preferably crystallized into particles having an average particle size of about 10µm or less.

In an alternative process a part of the surfactant is not added until after the product has cooled. This may help control the cyrstallization of the acyl derivative.

The present compositions are used in a conventional manner for cleaning hair. From about 0.1g to about 10g of a composition is applied to hair that has been wetted, generally with water, worked through the hair and then rinsed out.

The following Examples further describe and demonstrate the preferred embodiments within the scope of the present invention.

## EXAMPLES I - IV

The following compositions are representative of the present invention.

| | Weight % | | | |
|---|---|---|---|---|
| Component: | I | II | III | IV |
| ZPT[1] | 1 | 1 | 1 | 1 |
| TMPP[2] | 1 | - | 1 | 0.5 |
| CPIB[3] | - | 1 | - | 0.5 |
| Ammonium Lauryl Sulfate | 15 | 15 | 3.2 | 13.5 |
| Ammonium Laureth-3 Sulfate | 4 | 4 | 14 | 4 |
| PDMS[4] | 2 | 1 | 3 | 1.5 |
| Ethylene Glycol Distearate | 2 | 2 | 2 | 2 |
| Cocamide MEA | 1.5 | 1.5 | 1.5 | 1.5 |
| Cetyl Alcohol | 0.3 | 0.63 | 0.42 | 0.42 |
| Tricetyl Ammonium Chloride | 0.50 | 0.50 | 0.50 | 0.50 |
| Sodium Hydroxide | 0.175 | 0.175 | 0.15 | 0.15 |
| Stearyl Alcohol | 0.27 | 0.27 | 0.18 | 0.18 |
| Sodium Chloride | 0.2 | 0.2 | 0.22 | 0.21 |
| Sodium Citrate | 0.0038 | 0.0038 | 0.05 | 0.05 |
| Preservative, Dye Perfume and Water | ---- to 100% ---- | | | |
| pH | 4.6 | 4.6 | 4.6 | 4.6 |

[1]Zinc pyridinethione in platelet form having a mean sphericity of less than about 0.65 and a median particle size of about 18μm.
[2]1-hydroxy-4-methyl-6-(2,4,4-trimethylpentyl)-2-(1H)-pyridone, triethanolamine salt
[3]1-(p-chlorophenoxy)-1-(1H-imidazol-1-yl)-3,3-dimethyl-2-butanone
[4]Polydimethylsiloxane from General Electric having a viscosity of $10^6$ cps.

The compositions of Example I to IV combine excellent hair conditioning effectiveness, antidandruff efficacy and product stability.

## Claims

1. An antidandruff shampoo composition comprising:
   (a) from about 5% to about 70% by weight of a synthetic surfactant;
   (b) from about 0.01% to about 10% by weight of a dispersed, insoluble non-volatile silicone;
   (c) from about 0.5% to about 5% by weight of a long chain ($C_{16}$-$C_{22}$) acyl derivative or a long chain ($C_{16}$-$C_{22}$) amide oxide;
   (d) from about 0.1% to about 3% of a first antidandruff agent which is a 1-hydroxy-2-pyridinethione salt (I);
   (e) from about 0.1% to about 3% of a second antidandruff agent selected from
i) hydroxypyridones having the general formula II, or a salt thereof:

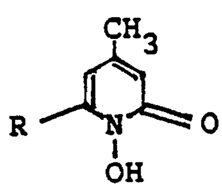

II

wherein R is an aliphatic or aromatic moiety having a $\pi$ factor of at least 1.3;
ii) imidazolyl ketone derivatives of the general formula III

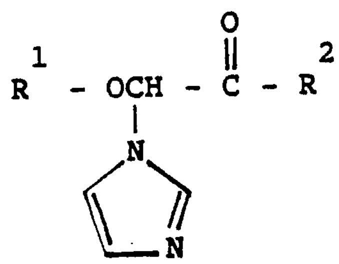

III

wherein $R^1$ and $R^2$ are independently selected from optionally substituted alkyl, alkenyl, alkynyl, cycloalkyl, aryl and alkaryl groups, and
iii) mixtures of (i) and (ii); and
f) water

2. A shampoo compaction awarding to claim 1 wherein the 1-hydroxy-2-pyridinethione salt (I) has a mean sphericity of less than about 0.65 and a median diameter of at least about 2μm.

3. A shampoo composition according to Claim 1 or 2 wherein the surfactant is selected from anionic surfactants, nonionic surfactants, zwitterionic surfactants, amphoteric surfactants and mixtures thereof.

4. A shampoo composition according to any of Claims 1 to 3 wherein the long chain acyl derivative or amine oxide is selected from ethylene glycol long chain esters, alkanolamides of long chain fatty acids, long chain esters of long chain fatty acids, glyceryl long chain esters, long chain esters of long chain alkanolamides, long chain alkyl dimethyl amide oxides and mixtures thereof.

5. A shampoo composition according to any of Claims 1 to 4 wherein the pyridinethione salt is a zinc salt.

6. A shampoo composition according to any of Claims 1 to 5 wherein the non-volatile silicone is selected from polydimethylsiloxanes having viscosities of from about 5 to about 100,000 centistokes at 25 °C, polypropylene oxide modified dimethylsiloxanes and silicone gums.

7. A shampoo composition according to any of Claims 1 to 6 wherein in formula II, R is selected from linear and branched $C_3$-$C_{11}$ alkyl and alkenyl groups, $C_5$-$C_8$ cycloalkyl groups and aryl groups.

8. A shampoo composition according to Claim 7 wherein R is 2,4,4-trimethylpentyl.

9. A shampoo composition according to any of Claims 1 to 8 wherein in formula III, $R^1$ is an optionally halo-substituted aryl or $C_1$-$C_4$ alkaryl group and $R^2$ is a straight chain or branched alkyl or alkaryl group having up to 8 carbon atoms.

10. A shampoo composition according to Claim 9 wherein the imidazolyl ketone is 1-(p-chlorophenoxy)-1-(1H-imidazol-1-yl)-3,3-dimethyl-2-butanone.

11. A shampoo composition according to any of Claims 1 to 10 which in addition contains a quaternary ammonium surfactant.

12. A shampoo composition according to any of Claims 1 to 11 wherein the first and second antidandruff agents are each present in a level of from about 0.5% to about 2%.

13. A shampoo composition according to any of Claims 1 to 12 wherein the acyl derivative is ethylene glycol distearate.

14. A shampoo composition according to any of Claims 1 to 13 wherein the shampoo composition has a pH of from about 4 to about 7.

15. A shampoo composition according to any of Claims 1 to 14 wherein the anionic surfactant is a mixture of alkyl sulfate and ethoxylated alkyl sulfate surfactants.

16. A shampoo composition according to Claim 15 wherein the anionic surfactant is a mixture of lauryl sulfate and ethoxylated lauryl sulfate surfactants in a molar ratio of less than about 1:3.